# EUROPEAN PATENT SPECIFICATION

(11) **EP 2 613 761 B1**
(45) Date of publication and mention of the grant of the patent: **18.07.2018**
(21) Application number: 11754381.9
(22) Date of filing: 06.09.2011
(51) Int. Cl.: A61K 8/90, A61Q 5/00, A61Q 5/12, A61K 8/73, A61K 8/34, A61K 8/41, A61K 8/84

(54) **COSMETIC COMPOSITION COMPRISING AT LEAST ONE CATIONIC POLYMER AND AT LEAST TWO CATIONIC SURFACTANTS**
KOSMETISCHE ZUSAMMENSETZUNG, DIE MINDESTENS EIN KATIONISCHES POLYMER UND MINDESTENS ZWEI KATIONISCHE TENSIDE ENTHÄLT.
COMPOSITION COSMETIQUE COMPRENANT AU MOINS UN POLYMÈRE CATIONIQUE ET AU MOINS DEUX TENSIOACTIFS CATIONIQUES.

(30) Priority: 17.09.2010 US 383788 P; 06.09.2010 FR 1057059
(43) Date of publication of application: 17.07.2013
(73) Proprietor: L'Oréal, 75008 Paris (FR)
(72) Inventor: BOURDIN, Claire, F-92300 Levallois Perret (FR); GILLES, Audrey, F-75015 Paris (FR); BEBOT, Cécile, F-92110 Clichy (FR)
(74) Representative: Leray, Noelle
(86) International application number: PCT/EP2011/065404
(87) International publication number: WO 2012/032055

(56) References cited:
- EP-A1- 1 378 227
- EP-A1- 1 634 569
- EP-A2- 1 023 888
- WO-A1-96/29980
- WO-A2-02/22085
- DE-C1- 19 735 865
- US-A- 5 034 219
- US-A- 5 726 137
- US-A1- 2007 010 408
- US-A1- 2007 292 380
- US-A1- 2010 152 691

## Description

The subject-matter of the present invention is a cosmetic composition comprising at least one cationic polymer and at least two surfactants.

It is well known that hair which has been sensitized, embrittled or indeed even damaged under the action of atmospheric agents or of mechanical and chemical treatments, such as dyeing operations, bleaching operations and/or perming operations, is often difficult to disentangle and to style and lacks softness. It also lacks sheen, the surface of the hair being detrimentally affected and sending back the light in a less homogeneous way.

The use has already been recommended, in compositions for the care of keratinous substances, such as the hair, of conditioning agents, in particular silicones, for facilitating the disentangling of the hair and for conferring softness and suppleness thereon. In the case in particular of leave-in care products, for the smoothness and sheen, it is generally silicone gums, in solution in volatile organic solvents, which are used.

However, the cosmetic advantages contributed by these conditioning agents are unfortunately also accompanied, on dry hair, by a greasy feel and a straggly visual appearance, which may be accompanied by faster regreasing of the hair between two shampooing operations.

Furthermore, the volatile organic solvents used to dissolve the silicone conditioning agents (liquid fatty ester, hydrocarbon oil of isododecane or isohexadecane type and/or silicone oil of cyclomethicone type) do not make it possible to overcome the abovementioned disadvantages.

In addition, in the case of anhydrous products in particular, these volatile solvents are present at a high concentration in the product and they have the disadvantage of having a sometimes disputed environmental profile.

DE 197 35 865 C1 (Goldwell GmbH) discloses an aqueous formulation useful as hair conditioner, treatment or leave-on lotion containing (a) quaternary ammonium compound(s) with 10-22 carbon (C) alk(en)yl group(s), (b) green tea extract and (c) a cationic polymer. US 5 726 137 (Colgate-Palmolive Company) discloses an hair conditioning shampoo comprising an aqueous dispersion of a water-insoluble lipid phase, an anionic hair cleansing surfactant, an amphoteric surfactant, at least one hair conditioning, film forming, polycationic polymer soluble in the aqueous dispersion and a water-insoluble, non-volatile hair conditioning silicone, the water-insoluble lipid phase comprising at least one lipid soluble, emulsifiable, monocationic, quaternary ammonium compound or amine solubilized in a water-insoluble long chain lipid compound. EP 1 378 227 A1 (L'Oréal Company) discloses a non-washing hair conditioning cosmetic composition comprising a quaternary ammonium silicone, a cationic surfactant, two cationic polymers and a non-associative nonionic thickening polymer. There thus exists a need to have available more effective conditioning compositions.

In some cases, clear compositions are also sought for.

The Applicant Company has discovered, surprisingly, that compositions comprising at least one cationic polymer and at least two specific surfactants make it possible to solve the problems touched on above.

More specifically, a subject-matter of the invention is a cosmetic composition comprising, in a cosmetically acceptable medium:
(i) one or more cationic polymers, at least one cationic polymer being a dimethyldiallylammonium chloride homopolymer, the cationic polymer or polymers being present in a content ranging from 0.01% to 15% by weight with respect to the total weight of the cosmetic composition,
(ii) two or more than two cationic surfactants, chosen from the quaternary ammonium salts corresponding to the formula (XI) as disclosed in the claims, the cationic surfactants being each present in a content ranging from 0.01% to 15% by weight with respect to the total weight of the cosmetic composition,
(iii) one or more polysaccharide thickeners,
(iv) one or more polyols with a molecular weight of less than 1000.

The term "molecular weight" is understood to mean the molecular weight expressed in g/mol.

Such a composition is easily distributed over the hair, resulting in clean, shiny, smooth and soft hair which is easily disentangled and the good cosmetic properties of which endure over time, even after several shampooing operations.

A further subject-matter of the invention is a method for the nontherapeutic cosmetic treatment of keratinous substances, in particular the hair, comprising the application, to the said substances, of a composition as described above.

### CATIONIC POLYMER

The composition according to the invention comprises one or more cationic polymers.

The term "cationic polymer" means a polymer that is positively charged when it is contained in the composition according to the invention. This polymer may bear one or more positive permanent charges or may contain one or more cationizable functions in the composition according to the invention.

At least one of the cationic polymers of the composition is a dimethyldiallylammonium chloride homopolymer sold under the name Merquat 100 by Nalco (and its homologues of low weight-average molecular weights).

The composition according to the invention can also comprise one or more other cationic polymer(s). The other cationic polymer(s) that may be used as conditioning agents according to the present invention are preferably chosen from polymers comprising primary, secondary, tertiary and/or quaternary amine groups forming part of the polymer chain or directly attached thereto, and having a molecular weight of between 500 and about 5 000 000 and preferably between 1000 and 3 000 000.

When the conditioning agent is a cationic polymer, it is preferably chosen from those that contain units comprising primary, secondary, tertiary and/or quaternary amine groups that may either form part of the main polymer chain, or may be borne by a side substituent directly attached thereto.

Among the cationic polymers that may be mentioned more particularly are polymers of the polyamine, polyaminoamide and polyquaternary ammonium type. These are known products. They are described, for example, in French Patents 2 505 348 and 2 542 997.

Among these polymers, mention may be made of:
(1) homopolymers or copolymers that are derived from acrylic or methacrylic esters or amides and comprise at least one of the units of the following formulae: 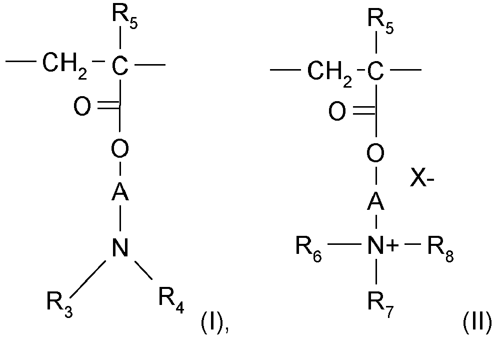 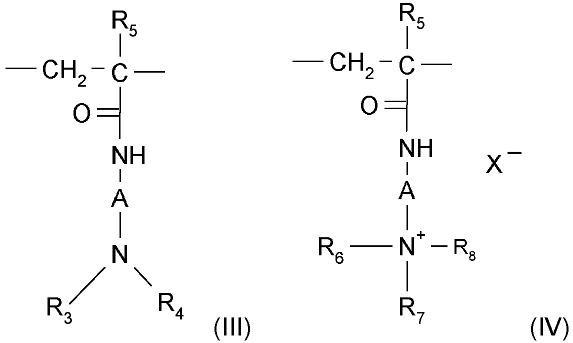 in which:
   R3 and R4, which may be identical or different, represent hydrogen or an alkyl group containing from 1 to 6 carbon atoms and preferably methyl or ethyl;
   R5, which may be identical or different, denote a hydrogen atom or a CH₃ radical;
   A, which may be identical or different, represent a linear or branched alkyl group of 1 to 6 carbon atoms, preferably 2 or 3 carbon atoms, or a hydroxyalkyl group of 1 to 4 carbon atoms;
   R6, R7 and R8, which may be identical or different, represent an alkyl group containing from 1 to 18 carbon atoms or a benzyl radical and preferably an alkyl group containing from 1 to 6 carbon atoms;
   X denotes an anion derived from a mineral or organic acid, such as a methosulphate anion or a halide such as chloride or bromide.

   The copolymers of the family (1) can also contain one or more units deriving from comonomers that may be chosen from the family of acrylamides, methacrylamides, diacetone acrylamides, acrylamides and methacrylamides substituted on the nitrogen with lower (C1-C4) alkyls, acrylic or methacrylic acids or esters thereof, vinyllactams such as vinylpyrrolidone or vinylcaprolactam, and vinyl esters.
   Thus, among these copolymers of the family (1), mention may be made of:
   - copolymers of acrylamide and of dimethylaminoethyl methacrylate quaternized with dimethyl sulphate or with a methyl halide, such as the product sold under the name Hercofloc by the company Hercules,
   - the copolymers of acrylamide and of methacryloyloxyethyltrimethylammonium chloride described, for example, in Patent Application EP-A-080 976 and sold under the name Bina Quat P 100 by the company Ciba Geigy,
   - the copolymer of acrylamide and of methacryloyloxyethyltrimethylammonium methosulphate sold under the name Reten by the company Hercules,
   - quaternized or non-quaternized vinylpyrrolidone/dialkylaminoalkyl acrylate or methacrylate copolymers, such as the products sold under the name Gafquat by the company ISP, such as, for example, Gafquat 734 or Gafquat 755, or alternatively the products known as Copolymer 845, 958 and 937. These polymers are described in detail in French Patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers sold in particular under the name Styleze CC 10 by ISP,
   - quaternized vinylpyrrolidone/dimethylaminopropylmethacrylamide copolymers such as the product sold under the name Gafquat HS 100 by the company ISP, and
   - crosslinked polymers of methacryloyloxy(C1-C4)alkyltri(C1-C4)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, more particularly methylenebisacrylamide. A crosslinked acrylamide/ methacryloyloxyethyltrimethylammonium chloride copolymer (20/80 by weight) in the form of a dispersion containing 50% by weight of the said copolymer in mineral oil can be used more particularly. This dispersion is sold under the name Salcare® SC 92 by the company Ciba. A crosslinked methacryloyloxyethyltrimethylammonium chloride homopolymer, for example as a dispersion in mineral oil or in a liquid ester, can also be used. These dispersions are sold under the names Salcare® SC 95 and Salcare® SC 96 by the company Ciba.
(2) Polymers formed from piperazinyl units and divalent alkylene or hydroxyalkylene radicals containing straight or branched chains, optionally interrupted with oxygen, sulphur or nitrogen atoms or with aromatic or heterocyclic rings, and also the oxidation and/or quaternization products of these polymers. Such polymers are especially described in French Patents 2 162 025 and 2 280 361.
(3) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quaternized. Such polymers are especially described in French Patents 2 252 840 and 2 368 508.
(4) Polyaminoamide derivatives resulting from the condensation of polyalkylenepolyamines with polycarboxylic acids followed by alkylation with difunctional agents. Mention may be made, for example, of adipic acid/dialkylaminohydroxyalkyldialkylenetriamine polymers in which the alkyl radical contains from 1 to 4 carbon atoms and preferably denotes methyl, ethyl or propyl. Such polymers are especially described in French Patent 1 583 363.
   Among these derivatives, mention may be made more particularly of the adipic acid/dimethylaminohydroxypropyldiethylenetriamine polymers sold under the name Cartaretine F, F4 or F8 by the company Sandoz.
(5) The polymers obtained by reaction of a polyalkylenepolyamine containing two primary amine groups and at least one secondary amine group with a dicarboxylic acid chosen from diglycolic acid and saturated aliphatic dicarboxylic acids having from 3 to 8 carbon atoms. The mole ratio between the polyalkylenepolyamine and the dicarboxylic acid is between 0.8:1 and 1.4:1; the polyaminoamide resulting therefrom is reacted with epichlorohydrin in a mole ratio of epichlorohydrin relative to the secondary amine group of the polyaminoamide of between 0.5:1 and 1.8:1. Such polymers are described in particular in US Patents 3 227 615 and 2 961 347.
   Polymers of this type are sold in particular under the name Hercosett 57 by the company Hercules Inc. or alternatively under the name PD 170 or Delsette 101 by the company Hercules in the case of the adipic acid/epoxypropyldiethylenetriamine copolymer.
(6) Alkyldiallylamine or dialkyldiallylammonium cyclopolymers, such as homopolymers or copolymers comprising, as main constituent of the chain, units corresponding to the formulae (V) and (VI): 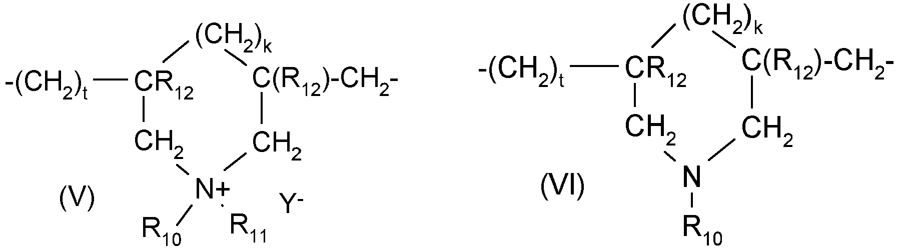 in which k and t are equal to 0 or 1, the sum k + t being equal to 1; R12 denotes a hydrogen atom or a methyl group; R10 and R11, independently of each other, denote an alkyl group having from 1 to 6 carbon atoms, a hydroxyalkyl group in which the alkyl group has preferably 1 to 5 carbon atoms, a lower (C1-C4) amidoalkyl group, or R10 and R11 may denote, jointly with the nitrogen atom to which they are attached, heterocyclic groups, such as piperidinyl or morpholinyl; Y⁻ is an anion such as bromide, chloride, acetate, borate, citrate, tartrate, bisulphate, bisulphite, sulphate or phosphate. These polymers are especially described in French Patent 2 080 759 and in its Certificate of Addition 2 190 406.
   R10 and R11, independently of one another, preferably denote an alkyl group having from 1 to 4 carbon atoms.
   Among the polymers defined above, mention may be made more particularly of the copolymers of diallyldimethylammonium chloride and of acrylamide, sold under the name Merquat 550.
(7) The diquaternary ammonium polymer containing repeating units corresponding to the formula: 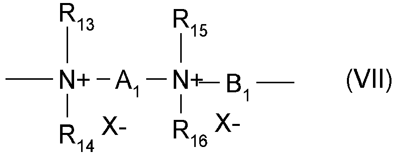 in which formula (VII):
   R13, R14, R15 and R16, which may be identical or different, represent aliphatic, alicyclic or arylaliphatic radicals containing from 1 to 20 carbon atoms, or lower hydroxyalkylaliphatic radicals, or else R13, R14, R15 and R16, together or separately, constitute, with the nitrogen atoms to which they are attached, heterocycles optionally containing a second heteroatom other than the nitrogen, or else R13, R14, R15 and R16 represent a linear or branched C1-C6 alkyl radical which is substituted by a nitrile, ester, acyl, amide or -CO-O-R17 -D or -CO-NH-R17-D group in which R17 is an alkylene and D is a quaternary ammonium group;
   A1 and B1 represent polymethylene groups containing from 2 to 20 carbon atoms, which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
   X⁻ denotes an anion derived from a mineral or organic acid;
   A1, R13 and R15 may form, with the two nitrogen atoms to which they are attached, a piperazine ring; moreover, if A1 denotes a saturated or unsaturated, linear or branched alkylene or hydroxyalkylene radical, B1 may also denote a group (CH₂)ₙ-CO-D-OC-(CH₂)ₚ-
   n and p are integers ranging from 2 to 20 approximately
   in which D denotes:
   a) a glycol residue of formula: -O-Z-O-, where Z denotes a linear or branched hydrocarbon-based radical or a group corresponding to one of the following formulae:

      -(CH₂-CH₂-O)ₓ-CH₂-CH₂-

      -[CH₂-CH(CH₃)-O]_{y}-CH₂-CH(CH₃)-

      where x and y denote an integer from 1 to 4, representing a defined and unique degree of polymerization, or any number from 1 to 4, representing an average degree of polymerization;
   b) a bis-secondary diamine residue such as a piperazine derivative;
   c) a bis-primary diamine residue of formula: -NH-Y-NH-, where Y denotes a linear or branched hydrocarbon-based radical, or alternatively the divalent radical

      -CH₂-CH₂-S-S-CH₂-CH₂-;
   d) a ureylene group of formula: -NH-CO-NH-;

Preferably, X⁻ is an anion such as chloride or bromide.

These polymers generally have a number-average molecular weight of between 1000 and 100 000.

Polymers of this type are described in particular in French Patents 2 320 330, 2 270 846, 2 316 271, 2 336 434 and 2 413 907 and US Patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.

Use may more particularly be made of the polymers which are composed of repeat units corresponding to the formula (VIII): 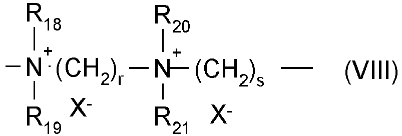 in which R18, R19, R20 and R21, which may be identical or different, denote an alkyl or hydroxyalkyl radical having from about 1 to 4 carbon atoms, r and s are integers varying from 2 to 20 approximately, and X⁻ is an anion derived from a mineral or organic acid.

One particularly preferred compound of formula (VIII) is that for which R18, R19, R20 and R21 represent a methyl radical and r = 3, s = 6 and X = Cl, which is called Hexadimethrine chloride according to the INCI (CTFA) nomenclature.
(8) Polyquaternary ammonium polymers composed of units of formula (IX): 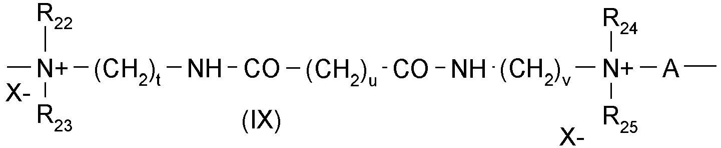 in which formula:
   R22, R23, R24 and R25, which may be identical or different, represent a hydrogen atom or a methyl, ethyl, propyl, β-hydroxyethyl, β-hydroxypropyl or -CH₂CH₂(OCH₂CH₂)ₚOH radical,
   in which p is equal to 0 or to an integer between 1 and 6, with the proviso that R22, R23, R24 and R25 do not simultaneously represent a hydrogen atom,
   t and u, which may be identical or different, are integers between 1 and 6,
   v is equal to 0 or to an integer between 1 and 34,
   X⁻ denotes an anion such as a halide,
   A denotes a radical of a dihalide or represents preferably -CH₂-CH₂-O-CH₂-CH₂-.

   Such compounds are described especially in Patent Application EP-A-122 324.
   Among these, mention may be made, for example, of the products Mirapol® A 15, Mirapol® AD1, Mirapol® AZ1 and Mirapol® 175, sold by the company Miranol.
(9) Quaternary polymers of vinylpyrrolidone and of vinylimidazole, such as, for example, the products sold under the names Luviquat® FC 905, FC 550 and FC 370 by the company BASF.
(10) Cationic polysaccharides, especially cationic celluloses and galactomannan gums.

Among cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives comprising quaternary ammonium groups described in French Patent 1 492 597. These polymers are also defined in the CTFA dictionary as quaternary ammoniums of hydroxyethylcellulose that has reacted with an epoxide substituted with a trimethylammonium group.

Cationic cellulose copolymers or cellulose derivatives grafted with a water-soluble quaternary ammonium monomer are described especially in US Patent 4 131 576, such as hydroxyalkylcelluloses, for instance hydroxymethyl-, hydroxyethyl- or hydroxypropyl- celluloses grafted, in particular, with a methacryloyloxyethyltrimethylammonium, methacrylamidopropyltrimethylammonium or dimethyldiallylammonium salt.

The cationic galactomannan gums are described more particularly in US Patents 3 589 578 and 4 031 307, in particular guar gums containing cationic trialkylammonium groups. Guar gums modified with a salt (e.g. chloride) of 2,3-epoxypropyltrimethylammonium are used, for example.

Other cationic polymers that may be used in the context of the invention are cationic proteins or cationic protein hydrolysates, polyalkyleneimines, in particular polyethyleneimines, polymers containing vinylpyridine or vinylpyridinium units, condensates of polyamines and of epichlorohydrin, quaternary polyureylenes and chitin derivatives.

The cationic proteins or protein hydrolysates are, in particular, chemically modified polypeptides bearing quaternary ammonium groups at the end of the chain, or grafted to the chain. Their molecular weight may vary, for example, from 1500 to 10 000 and in particular from 2000 to 5000 approximately. Among these compounds, mention may be made especially of:
- collagen hydrolysates carrying triethylammonium groups, such as the products sold under the name Quat-Pro E by the company Maybrook and referred to in the CTFA dictionary as Triethonium Hydrolyzed Collagen Ethosulfate;
- collagen hydrolysates bearing trimethylammonium chloride and trimethyl-stearylammonium chloride groups, which are sold under the name Quat-Pro S by the company Maybrook and are referred to in the CTFA dictionary as Steartrimonium Hydrolyzed Collagen;
- animal protein hydrolysates which bear trimethylbenzylammonium groups, such as the products sold under the name Crotein BTA by the company Croda and referred to in the CTFA dictionary as Benzyltrimonium hydrolyzed animal protein;
- protein hydrolysates bearing quaternary ammonium groups on the polypeptide chain, the said ammonium groups containing at least one alkyl radical having from 1 to 18 carbon atoms.

Among these protein hydrolysates, mention may be made, inter alia, of:
- Croquat L, in which the quaternary ammonium groups contain a C12 alkyl group;
- Croquat M, in which the quaternary ammonium groups contain C10-C18 alkyl groups;
- Croquat S, in which the quaternary ammonium groups contain a C18 alkyl group;
- Crotein Q, in which the quaternary ammonium groups contain at least one alkyl group having from 1 to 18 carbon atoms.

These various products are sold by the company Croda.

Other quaternized proteins or hydrolysates are, for example, those corresponding to the formula (X): 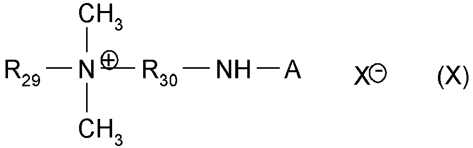 in which X⁻ is an anion of an organic or inorganic acid, A denotes a protein residue derived from collagen protein hydrolysates, R29 denotes a lipophilic group containing up to 30 carbon atoms, R30 represents an alkylene group having 1 to 6 carbon atoms. Mention may be made, for example, of the products sold by the company Inolex, under the name Lexein QX 3000, referred to in the CTFA dictionary as Cocotrimonium Collagen Hydrolysate.

Mention may also be made of quaternized plant proteins such as wheat, maize or soybean proteins: quaternized wheat proteins that may be mentioned include those sold by the company Croda under the names Hydrotriticum WQ or QM, referred to in the CTFA dictionary as Cocodimonium hydrolysed wheat protein, Hydrotriticum QL, referred to in the CTFA dictionary as Laurdimonium hydrolysed wheat protein, or else Hydrotriticum QS, referred to in the CTFA dictionary as Steardimonium hydrolysed wheat protein.

Among all the cationic polymers capable of being used in the context of the present invention, it is preferable to employ cationic cyclopolymers, in particular the dimethyldiallylammonium chloride homopolymers or copolymers sold under the names Merquat 100, Merquat 550 and Merquat S by the company Nalco, quaternary polymers of vinylpyrrolidone and of vinylimidazole, cationic polysaccharides and their mixtures.

The cationic polymer or polymers i) are present in a content ranging from 0.01% to 15% by weight, preferably ranging from 0.1% to 10% by weight and more preferentially ranging from 0.2% to 5% by weight, with respect to the total weight of the composition of the invention.

### CATIONIC SURFACTANT

The composition according to the invention comprises two or more specific cationic surfactants.

According to one alternative form, the composition comprises three specific surfactants. Generally, a cationic surfactant can carry one or more permanent positive charges or can comprise one or more functional groups which can form cations in the composition according to the invention. The cationic surfactants of the invention are those carrying one or more permanent positive charges, that is to say one or more quaternized nitrogen atoms. Two or more than two cationic surfactants of the present invention are chosen from:
- quaternary ammonium salts corresponding to the following general formula (XI): 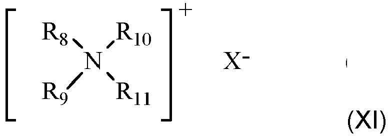 in which the R₈ to R₁₁ radicals, which can be identical or different, represent a linear or branched aliphatic radical comprising from 1 to 30 carbon atoms, at least one of the R₈ to R₁₁ radicals comprising from 8 to 30 carbon atoms, preferably from 12 to 24 carbon atoms. The aliphatic radicals can comprise heteroatoms, such as, in particular, oxygen, nitrogen or sulphur, and halogens.

The aliphatic radicals are, for example, chosen from alkyl, alkoxy, polyoxy(C₂-C₆)alkylene, alkylamide, (C-₁₂-C₂₂)alkylamido(C₂-C₆)alkyl, (C₁₂-C₂₂)alkyl acetate or hydroxyalkyl radicals comprising approximately from 1 to 30 carbon atoms; X⁻ is an anion chosen from the group of the halides, phosphates, acetates, lactates, (C₂-C₆)alkyl sulphates, or alkyl- or alkylarylsulphonates.

Preference is given, among the cationic surfactants of formula (XI) to tetraalkylammonium chlorides, such as, for example, dialkyldimethylammonium or alkyltrimethylammonium chlorides in which the alkyl radical comprises approximately from 12 to 22 carbon atoms, in particular behenyltrimethylammonium chloride, distearyldimethylammonium chloride, cetyltrimethylammonium chloride or benzyldimethylstearylammonium chloride.

It is preferable, among all the cationic surfactants which can be present in the composition according to formula (XI) of the invention, to choose cetyltrimethylammonium or behenyltrimethylammonium. The cationic surfactants ii) are present each in a content ranging from 0.01% to 15% by weight, preferably ranging from 0.1% to 10% by weight and more preferably ranging from 0.2% to 5% by weight, with respect to the total weight of the composition of the invention.

Preferably, the cationic polymer(s) (i)/cationic surfactants ratio by weight is less than 1. Better still, it is between 0.05 and 1.

### POLYSACCHARIDE THICKENER

The term "thickener" is understood to mean, within the meaning of the invention, a compound capable, by its presence, of increasing the viscosity of the medium by at least 50 cPs at 25°C and at a shear rate of 1 s⁻¹. Preferably, the thickening agent, at 1% in water or a 50/50 by weight water/alcohol mixture, exhibits a viscosity at 25°C and at a shear rate of 1 s⁻¹ of greater than 100 cPs.

These viscosities can be measured using in particular viscometers or rheometers having cone/plate geometry.

Polysaccharide thickeners are polymers which exhibit monosaccharides or disaccharides as base units. The polymers which can be used in the compositions according to the present invention are preferably chosen from guar gums and modified guar gums, celluloses, gellan gum and its derivatives.

Preferably, the polysaccharides of the invention are nonionic.

Guar gums are galactomannans composed of mannose and of galactose.

The term "modified guar gum" is understood to mean, within the meaning of the present patent application, guar gums alkylated by at least one C₁₋₈ alkyl group, guar gums hydroxyalkylated by at least one C₁₋₈ hydroxyalkyl group or guar gums acylated by at least one C₁₋₈ acyl group.

Preferably, they will be hydroxypropylated guar gums, such as that sold under the name Jaguar HP 105 by Rhodia.

Cellulose is a β-1,4-polyacetal of cellobiose, cellobiose being a disaccharide composed of two glucose molecules.

The celluloses which can be used in the compositions according to the present invention are preferably chosen from nonionic celluloses not comprising a hydrophobic chain and nonionic celluloses comprising one or more hydrophobic chains.

Preferably, the ionic celluloses used according to the invention are cellulose ethers. More preferably still, these celluloses are hydroxyalkylcelluloses and in particular hydroxyethylcelluloses or hydropropylcelluloses. They may or may not contain a fatty chain. Among the non-ionic celluloses that do not comprise a fatty chain, mention may be made of hydroxyethylcelluloses, hydropropylcelluloses, and hydroxypropylmethylcelluloses. One particularly suitable hydroxypropylmethylcellulose is Methocel F4M sold by Dow Chemicals (INCI name: Hydroxypropylmethylcellulose).

The celluloses modified with groups comprising one or more nonionic fatty chains that can be used according to the present invention are, in particular:
- hydroxyethylcelluloses, preferably nonionic hydroxyethylcelluloses, modified by groups comprising at least one fatty chain, such as alkyl, arylalkyl or alkylaryl groups, or their mixtures, and in which the alkyl groups are preferably C₈-C₂₂ alkyl groups, such as the product Natrosol Plus Grade 330 CS® (C₁₆ alkyls), sold by Aqualon, corresponding to the INCI name Cetylhydroxyethylcellulose, or the product Bermocoll EHM 100® sold by Berol Nobel,
- those modified with alkylphenyl polyalkylene glycol ether groups, such as the product Amercell Polymer HM-1500® (nonylphenyl polyethylene glycol (15) ether) sold by Amerchol that corresponds to the INCI name Nonoxynyl hydroxyethylcellulose.

Gellan gum is a polysaccharide produced by aerobic fermentation of *Sphingomonas elodea,* more commonly known as *Pseudomonas elodea.* This linear polysaccharide is composed of the sequence of the following monosaccharides: D-glucose, D-glucuronic acid and L-rhamnose. In the native state, gellan gum is highly acylated.

The gellan gum preferably used in the compositions according to the present invention is an at least partially deacylated gellan gum. This at least partially deacylated gellan gum is obtained by a high-temperature alkaline treatment. A KOH or NaOH solution will, for example, be used.

The purified gellan gum sold under the trade name Kelcogel® by Kelco is suitable for preparing the compositions according to the invention.

The derivatives of gellan gum are all products obtained by carrying out conventional chemical reactions, such as in particular esterifications or addition of a salt of an organic or inorganic acid.

Use is made, as derivative of gellan gum, for example, of welan gum. Welan gum is a gellan gum modified by fermentation by means of *Alcaligenes* strain ATCC 31 555. Welan gum has a repeating pentasaccharide structure formed of a main chain composed of D-glucose, D-glucuronic acid and L-rhamnose units to which a pendant unit of L-rhamnose or of L-mannose is grafted.

The welan gum sold under the trade name Kelco Crete® by Kelco is suitable for preparing the compositions according to the invention.

Mention may be made, as other saccharide thickening polymers which can be used according to the invention, of starches and their derivatives.

The concentration of polysaccharide thickeners used in the compositions according to the present invention is between 0.05 and 10% by weight, preferably between 0.1 and 5% by weight and more preferably still between 0.5 and 3% by weight, with respect to the total weight of the composition.

### POLYOL

The polyols which can be used in the composition according to the present invention are compounds composed of a hydrocarbon-based chain optionally interrupted by one or more oxygen atoms and carrying at least two free hydroxyl groups and with a molecular weight of less than 1000 .

The hydrocarbon-based chain can be linear or branched and saturated or unsaturated.

Preferably, the hydrocarbon-based chain comprises from 3 to 50 carbon atoms and better still from 3 to 20 carbon atoms.

In an alternative form of the invention, the hydrocarbon-based chain is saturated.

Very particularly, the polyols of the invention comprise 2 or 3 free hydroxyl functional groups.

The free hydroxyl functional groups can be carried by contiguous carbon atoms or the carbon atoms carrying them can be separated by one or more other carbon atoms.

Use is preferably made, among these polyols, of 1,2-propanediol (or propylene glycol), 1,3-propanediol, glycerol, 3-(2-ethylhexyl)oxy-1,2-propanediol (or ethylhexylglycerin), 3-methyl-1,3,5-pentanetriol, 1,2,4-butanetriol, 1,5-pentanediol, 2-methyl-1,3-propanediol, 1,3-butanediol, 3-methyl-1,5-pentanediol, neopentyl glycol (2,2-dimethyl-1,3-propanediol), isoprene glycol (3-methyl-1,3-butanediol) and hexylene glycol (2-methyl-2,4-pentanediol), diethylene glycol, dipropylene glycol, tripropylene glycol or polyethylene glycols of formula H(OCH₂CH₂)ₙOH with n ranging from 4 to 16.

Preferably, the molecular weight (MW) of the polyol is between 75 and 500. More preferably still, the molecular weight is between 90 and 350.

The polyol or polyols can be present in the composition at a content of between 0.1 and 30% by weight, preferably between 0.5 and 20% by weight and more preferably still between 1 and 15% by weight of the total weight of the cosmetic composition.

### MEDIUM

The composition according to the invention can additionally comprise one or more conventional additives well known in the art other than the compounds defined above. Mention may be made, as examples of additives which can be used according to the invention, of silanes, anionic surfactants, amphoteric or zwitterionic surfactants, nonionic surfactants, proteins, protein hydrolysates, vitamins, reducing agents, plasticizers, softeners, antifoaming agents, moisturizing agents, pigments, clays, inorganic fillers, UV screening agents, abrasive agents (pumice, apricot kernel powder), inorganic colloids, peptizing agents, solubilizing agents, fragrances, preservatives, pearlescent agents, propellants, inorganic or organic thickeners, antidandruff agents (for example, zinc pyrithione, octopirox, selenium sulphide, ellagic acid and derivatives), agents for combating hair loss or agents for promoting hair regrowth; these additives being other than the compounds defined above.

A person skilled in the art will take care to select the optional additive(s) and the amount thereof such that they do not harm the properties of the compositions of the present invention.

The additive(s) are generally present in the composition according to the invention in an amount ranging from 0 to 20% by weight relative to the total weight of the composition.

The compositions according to the invention can be provided in all the formulation forms conventionally used for a topical application and in particular in the form of aqueous or aqueous/alcoholic solutions, of oil-in-water (O/W), water-in-oil (W/O) or multiple (triple: W/O/W or O/W/O) emulsions, of aqueous gels or of dispersions of a fatty phase in an aqueous phase using spherules, it being possible for these spherules to be polymeric nanoparticles, such as nanospheres and nanocapsules, or lipid vesicles of ionic and/or nonionic type (liposomes, niosomes or oleosomes). These compositions are prepared according to the usual methods.

In addition, the compositions used according to the invention can be more or less fluid and can have the appearance of a white or coloured cream, of an ointment, of a milk, of a lotion, of a serum, of a paste or of a foam. They can optionally be applied to the keratinous substances in the aerosol form. They can also be provided in the solid form and for example in the stick form.

Of course, the person skilled in the art will take care to choose the optional compound or compounds to be added to the composition according to the invention so that the advantageous properties intrinsically attached to the composition in accordance with the invention are not, or not substantially, detrimentally affected by the envisaged addition or additions.

Another subject-matter of the invention is a method for the cosmetic treatment of keratinous substances, in particular keratinous fibres and especially human keratinous fibres, such as the hair, comprising the application of a composition according to the invention to the said keratinous substances and in particular the keratinous fibres. After an optional leave-in time preferably ranging from 1 to 30 minutes, the composition is subsequently preferably rinsed out with water. Preferably, the said method is intended for the conditioning of the said keratinous fibres.

According to a specific form of the invention, the method for the treatment of keratinous fibres can comprise a stage of heating the keratinous fibres during or after application of the composition according to the invention.

The keratinous fibres can, for example, be heated by means of an iron, of a liquid water/steam mixture or of a drying hood or hairdryer.

The application of the composition of the invention can also be preceded or followed by the application of an acid composition (for example, pH 2 to 6) or basic composition (for example, pH 8 to 12).

Another subject-matter of the invention is the use of a composition according to the invention for caring for keratinous substances, in particular keratinous fibres and especially human keratinous fibres, such as the hair.

The examples which follow are intended to illustrate the invention without, however, exhibiting a limiting nature.

### Example 1: Leave-in serum

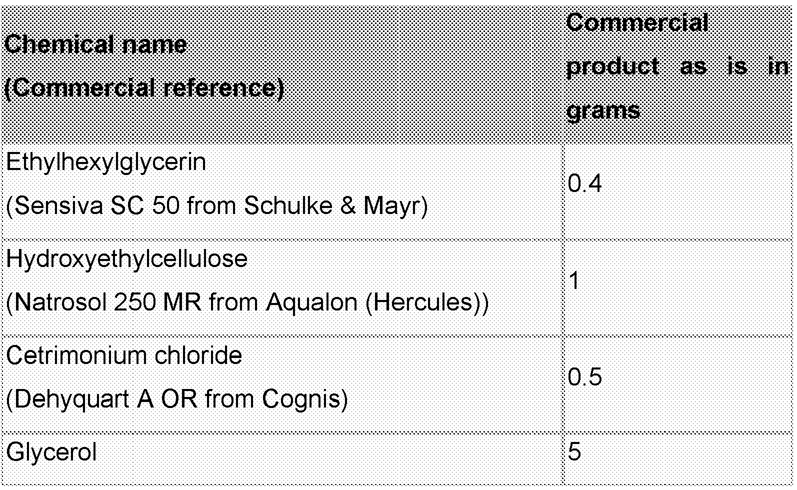

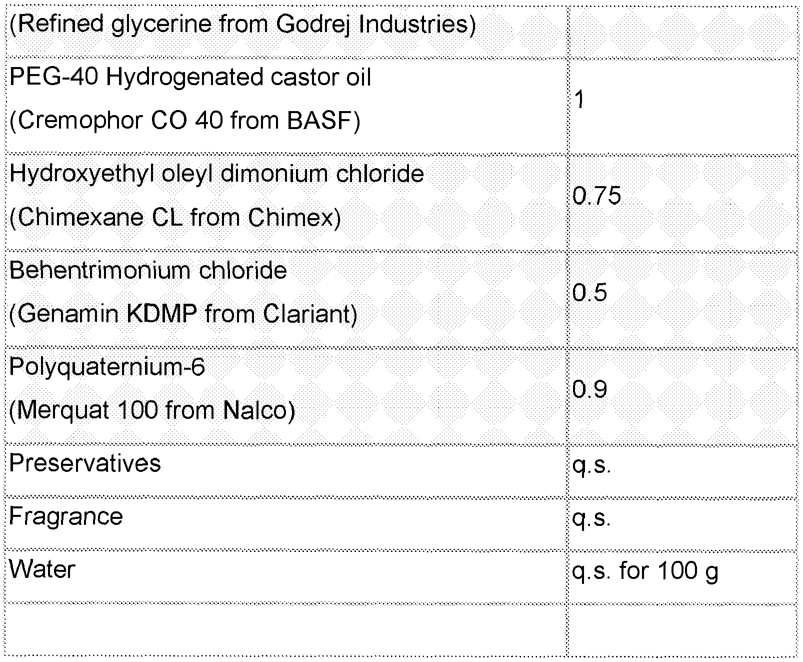

This serum is clear. Applied to clean hair, it confers thereon, after drying, sheen, smoothness and softness. The hair is easily disentangled.

### Example 2: Leave-in serum (not part of the invention as claimed)

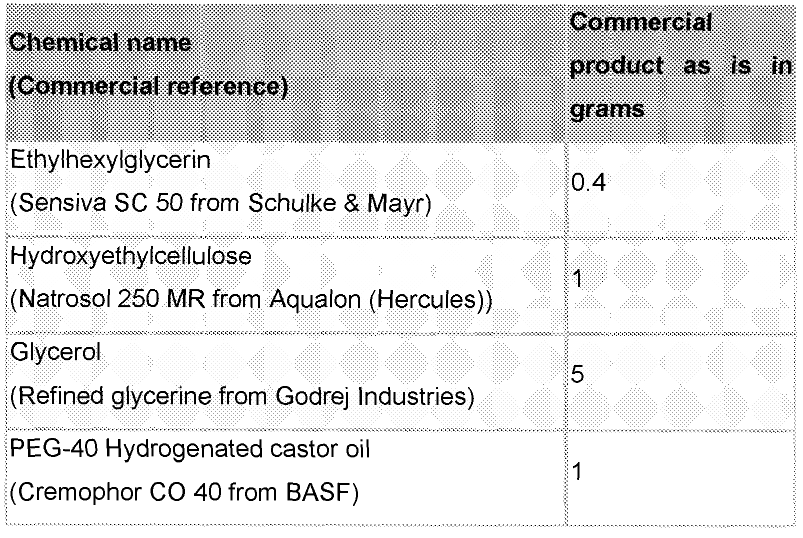

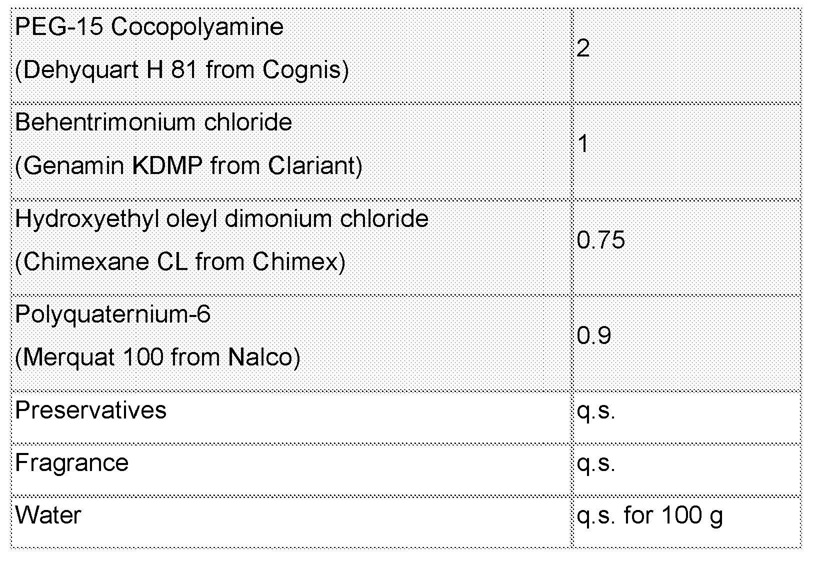

### Example 3: Leave-in serum

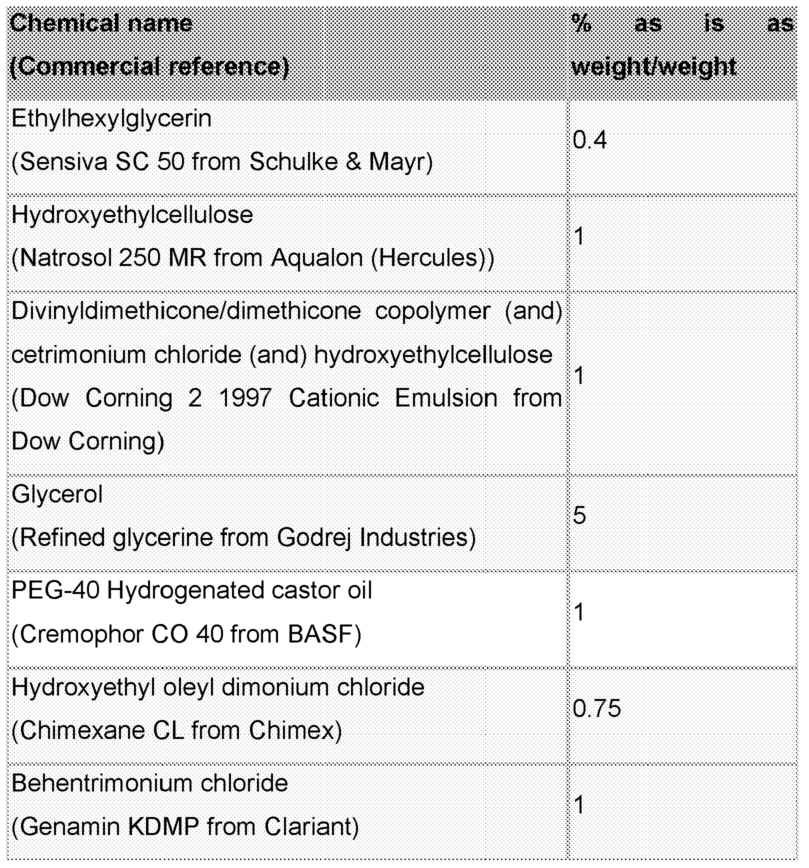

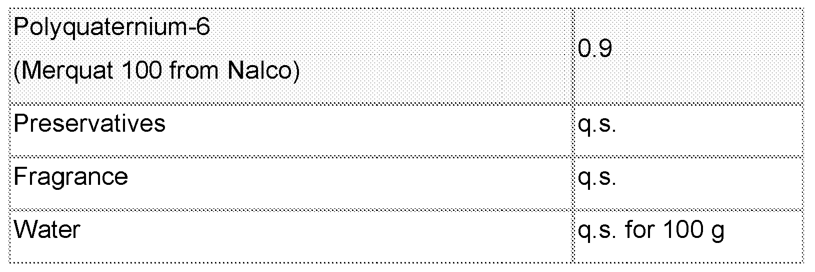

## Claims

1. Cosmetic composition comprising:
(i) one or more cationic polymers, at least one cationic polymer being a dimethyldiallylammonium chloride homopolymer, the cationic polymer or polymers being present in a content ranging from 0.01% to 15% by weight with respect to the total weight of the cosmetic composition,
(ii)two or more than two cationic surfactants chosen from the quaternary ammonium salts corresponding to the following general formula (XI): 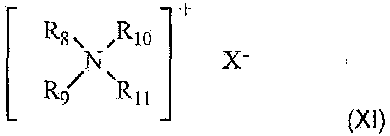 in which the R₈ to R₁₁ radicals, which are identical or different, represent a linear or branched aliphatic radical comprising from 1 to 30 carbon atoms and which can comprise heteroatoms, at least one of the R₈ to R₁₁ radicals comprising from 8 to 30 carbon atoms, the cationic surfactants being each present in a content ranging from 0.01% to 15% by weight with respect to the total weight of the cosmetic composition, X- is an anion chosen from the group of the halides, phosphates, acetates, lactates, (C₂-C₆) alkyl sulphates, or alkyl- or alkylarylsulphonates;
(iii) one or more polysaccharide thickeners,
(iv) one or more polyols with a molecular weight of less than 1000.

2. Composition according to the preceding claim, **characterized in that** the cationic polymer or polymers are present in a content ranging from 0.1% to 10% by weight and more preferably ranging from 0.2% to 5% by weight, with respect to the total weight of the cosmetic composition.

3. Composition according to either one of Claims 1 and 2, **characterized in that** the cationic surfactant or surfactants are each present in a content ranging from 0,1% to 10% by weight and more preferably ranging from 0.2% to 5% by weight, with respect to the total weight of the cosmetic composition.

4. Cosmetic composition according to any one of the preceding claims, in which the polysaccharide thickeners are nonionic.

5. Cosmetic composition according to any one of the preceding claims, in which the polysaccharide thickeners are chosen from celluloses and in particular from cellulose ethers.

6. Cosmetic composition according to any one of the preceding claims, **characterized in that** the polyols are compounds composed of a hydrocarbon-based chain comprising from 3 to 50 carbon atoms, better still from 3 to 20 carbon atoms, and optionally interrupted by one or more oxygen atoms and carrying at least two free hydroxyl groups.

7. Cosmetic composition according to any one of the preceding claims, **characterized in that** the polyol is chosen from 1,2-propanediol (or propylene glycol), 1,3-propanediol, glycerol, 3-(2-ethylhexyl)oxy-1,2-propanediol (or ethylhexylglycerin), 3-methyl-1,3,5-pentanetriol, 1,2,4-butanetriol, 1,5-pentanediol, 2-methyl-1,3-propanediol, 1,3-butanediol, 3-methyl-1,5-pentanediol, neopentyl glycol (2,2-dimethyl-1,3-propanediol), isoprene glycol (3-methyl-1,3-butanediol) and hexylene glycol (2-methyl-2,4-pentanediol), diethylene glycol, dipropylene glycol, tripropylene glycol or polyethylene glycols of formula H(OCH₂CH₂)ₙOH with n ranging from 4 to 16.

8. Cosmetic composition according to any one of the preceding claims, **characterized in that** the molecular weight (MW) of the polyol is between 75 and 500 and more preferably still between 90 and 350.

9. Cosmetic composition according to any one of the preceding claims, **characterized in that** the polyol or polyols are present in the composition at a content of between 0.1 and 30% by weight, preferably between 0.5 and 20% by weight and more preferably still between 1 and 15% by weight of the total weight of the cosmetic composition.

10. Method for the nontherapeutic cosmetic treatment of keratinous substances, in particular keratinous fibres and especially human keratinous fibres, such as the hair, comprising the application, to the said substances, of a composition as described in one of Claims 1 to 9.

11. Use of a composition according to one of Claims 1 to 9for the conditioning of keratinous substances, in particular keratinous fibres and especially human keratinous fibres, such as the hair.

## Patentansprüche

1. Kosmetische Zusammensetzung, umfassend:
(i) ein oder mehrere kationische Polymere, wobei es sich bei mindestens einem kationischen Polymer um ein Dimethyldiallylammoniumchlorid-Homopolymer handelt, wobei das kationische Polymere bzw. die kationischen Polymere in einem Gehalt im Bereich von 0,01 bis 15 Gew.-%, bezogen auf das Gesamtgewicht der kosmetischen Zusammensetzung, vorliegt bzw. vorliegen,
(ii) zwei oder mehr als zwei kationische Tenside, die aus den quaternären Ammoniumsalzen der folgenden allgemeinen Formel (XI): 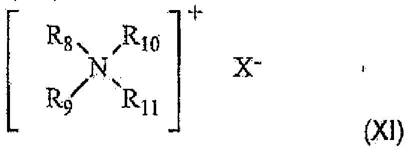 ausgewählt sind, wobei die Reste R₈ bis R₁₁, die gleich oder verschieden sind, für einen linearen oder verzweigten aliphatischen Rest mit 1 bis 30 Kohlenstoffatomen und gegebenenfalls Heteroatomen steht, wobei mindestens einer der Reste R₈ bis R₁₁ 8 bis 30 Kohlenstoffatome aufweist, wobei die kationische Tenside jeweils in einem Gehalt im Bereich von 0,01 bis 15 Gew.-%, bezogen auf das Gesamtgewicht der kosmetischen Zusammensetzung, vorliegen,
x⁻ für ein Anion aus der Gruppe der Halogenide, Phosphate, Acetate, Lactate, (C₂-C₆)Alkylsulfate oder Alkyl- oder Alkylarylsulfonate steht;
(iii) einen oder mehrere Polysaccharid-Verdicker,
(iv) ein oder mehrere Polyole mit einem Molekulargewicht von weniger als 1000.

2. Zusammensetzung nach dem vorhergehenden Anspruch, **dadurch gekennzeichnet, dass** das kationische Polymer bzw. die kationischen Polymere in einem Gehalt im Bereich von 0,1 bis 10 Gew.-% und weiter bevorzugt im Bereich von 0,2 bis 5 Gew.-%, bezogen auf das Gesamtgewicht der kosmetischen Zusammensetzung, vorliegt bzw. vorliegen.

3. Zusammensetzung nach einem der Ansprüche 1 und 2, **dadurch gekennzeichnet, dass** das kationische Tenside bzw. die kationische Tenside jeweils in einem Gehalt im Bereich von 0,1 bis 10 Gew.-% und weiter bevorzugt im Bereich von 0,2 bis 5 Gew.-%, bezogen auf das Gesamtgewicht der kosmetischen Zusammensetzung, vorliegt bzw. vorliegen.

4. Kosmetische Zusammensetzung nach einem der vorhergehenden Ansprüche, wobei die Polysaccharid-Verdicker nichtionisch sind.

5. Kosmetische Zusammensetzung nach einem der vorhergehenden Ansprüche, wobei die Polysaccharid-Verdicker aus Cellulosen und insbesondere aus Celluloseethern ausgewählt sind.

6. Kosmetische Zusammensetzung nach einem der vorhergehenden Ansprüche, **dadurch gekennzeichnet, dass** es sich bei den Polyolen um Verbindungen handelt, die aus einer auf Kohlenwasserstoff basierenden Kette, die 3 bis 50 Kohlenstoffatome und noch besser 3 bis 20 Kohlenstoffatome aufweist und gegebenenfalls durch ein oder mehrere Sauerstoffatome unterbrochen ist und mindestens zwei freie Hydroxylgruppen trägt.

7. Kosmetische Zusammensetzung nach einem der vorhergehenden Ansprüche, **dadurch gekennzeichnet, dass** das Polyol aus 1,2-Propandiol (oder Propylenglykol), 1,3-Propandiol, Glycerin, 3-(2-Ethylhexyl)oxy-1,2-propandiol (oder Ethylhexylglycerin), 3-Methyl-1,3,5-pentantriol, 1,2,4-Butantriol, 1,5-Pentandiol, 2-Methyl-1,3-propandiol, 1,3-Butandiol, 3-Methyl-1,5-pentandiol, Neopentylglykol (2,2-Dimethyl-1,3-propandiol), Isoprenglykol (3-Methyl-1,3-butandiol) und Hexylenglykol (2-Methyl-2,4-pentandiol), Diethylenglykol, Dipropylenglykol, Tripropylenglykol oder Polyethylenglykolen der Formel H(OCH₂CH₂)ₙOH mit n im Bereich von 4 bis 16 ausgewählt ist.

8. Kosmetische Zusammensetzung nach einem der vorhergehenden Ansprüche, **dadurch gekennzeichnet, dass** das Molekulargewicht (MW) des Polyols zwischen 75 und 500 und noch weiter bevorzugt zwischen 90 und 350 liegt.

9. Kosmetische Zusammensetzung nach einem der vorhergehenden Ansprüche, **dadurch gekennzeichnet, dass** das Polyol bzw. die Polyole in der Zusammensetzung in einem Gehalt zwischen 0,1 und 30 Gew.-%, vorzugsweise zwischen 0,5 und 20 Gew.-% und noch weiter bevorzugt zwischen 1 und 15 Gew.-%, bezogen auf das Gesamtgewicht der kosmetischen Zusammensetzung, vorliegen.

10. Verfahren zur nichttherapeutischen kosmetischen Behandlung von Keratinsubstanzen, insbesondere Keratinfasern und speziell menschlichen Keratinfasern, wie dem Haar, bei dem man auf die Substanzen eine Zusammensetzung gemäß einem der Ansprüche 1 bis 9 aufbringt.

11. Verwendung einer Zusammensetzung nach einem der Ansprüche 1 bis 9 zur Konditionierung von Keratinsubstanzen, insbesondere Keratinfasern und speziell menschlichen Keratinfasern, wie dem Haar.

## Revendications

1. Composition cosmétique, comprenant :
(i) un ou plusieurs polymères cationiques, au moins un polymère cationique étant un homopolymère de chlorure de diméthyldiallylammonium, le polymère ou les polymères cationiques étant présents selon une teneur allant de 0,01% à 15% en poids par rapport au poids total de la composition cosmétique ;
(ii) deux, ou plus de deux, agents tensioactifs cationiques choisis parmi les sels d'ammonium quaternaire correspondant à la formule générale (XI) suivante : 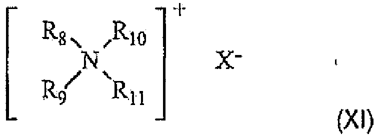 où les radicaux R₈ à R₁₁, qui sont identiques ou différents, représentent un radical aliphatique linéaire ou ramifié comprenant de 1 à 30 atomes de carbone et pouvant comprendre des hétéroatomes, au moins l'un parmi les radicaux R₈ à R₁₁ comprenant de 8 à 30 atomes de carbone, les agents tensioactifs cationiques étant chacun présents selon une teneur allant de 0,01% à 15% en poids par rapport au poids total de la composition cosmétique ; X⁻ est un anion choisi dans le groupe constitué par les halogénures, les phosphates, les acétates, les lactates, les (C₂-C₆) alkylsulfates, ou les alkyl- ou alkylarylsulfonates ;
(iii) un ou plusieurs épaississants à base de polysaccharides ;
(iv) un ou plusieurs polyols ayant un poids moléculaire inférieur à 1000.

2. Composition selon la revendication précédente, **caractérisée en ce que** le polymère ou les polymères cationiques sont présents selon une teneur allant de 0,1% à 10% en poids, et plus préférablement allant de 0,2% à 5% en poids, par rapport au poids total de la composition cosmétique.

3. Composition selon l'une ou l'autre parmi les revendications 1 et 2, **caractérisée en ce que** l'agent tensioactif ou les agents tensioactifs cationiques sont chacun présents selon une teneur allant de 0,1% à 10% en poids, et plus préférablement allant de 0,2% à 5% en poids, par rapport au poids total de la composition cosmétique.

4. Composition cosmétique selon l'une quelconque des revendications précédentes, dans laquelle les épaississants à base de polysaccharides sont non ioniques.

5. Composition cosmétique selon l'une quelconque des revendications précédentes, dans laquelle les épaississants à base de polysaccharides sont choisis parmi les celluloses et en particulier parmi les éthers de cellulose.

6. Composition cosmétique selon l'une quelconque des revendications précédentes, **caractérisée en ce que** les polyols sont des composés constitués d'une chaîne hydrocarbonée comprenant de 3 à 50 atomes de carbone, mieux encore de 3 à 20 atomes de carbone, et éventuellement interrompue par un ou plusieurs atomes d'oxygène et portant au moins deux groupements hydroxyle libres.

7. Composition cosmétique selon l'une quelconque des revendications précédentes, **caractérisée en ce que** le polyol est choisi parmi le 1,2-propanediol (ou propylène glycol), le 1,3-propanediol, le glycérol, le 3-(2-éthylhexyl)oxy-1,2-propanediol (ou éthylhexylglycérol), le 3-méthyl-1,3,5-pentanetriol, le 1,2,4-butanetriol, le 1,5-pentanediol, le 2-méthyl-1,3-propanediol, le 1,3-butanediol, le 3-méthyl-1,5-pentanediol, le néopentyl-glycol (2,2-diméthyl-1,3-propanediol), l'isoprène glycol (3-méthyl-1,3-butanediol) et l'hexylène glycol (2-méthyl-2,4-pentanediol), le diéthylène glycol, le dipropylène glycol, le tripropylène glycol ou les polyéthylène glycols de formule H(OCH₂CH₂)ₙOH, n allant de 4 à 16.

8. Composition cosmétique selon l'une quelconque des revendications précédentes, **caractérisée en ce que** le poids moléculaire (PM) du polyol est compris entre 75 et 500 et encore plus préférablement entre 90 et 350.

9. Composition cosmétique selon l'une quelconque des revendications précédentes, **caractérisée en ce que** le polyol ou les polyols sont présents dans la composition selon une teneur comprise entre 0,1 et 30% en poids, préférablement entré 0,5 et 20% en poids, et encore plus préférablement entre 1 et 15% en poids du poids total de la composition cosmétique.

10. Méthode de traitement cosmétique non thérapeutique de substances kératiniques, en particulier de fibres kératiniques et notamment de fibres kératiniques humaines, telles que les cheveux, comprenant l'application auxdites substances d'une composition telle que décrite selon l'une des revendications 1 à 9.

11. Utilisation d'une composition selon l'une des revendication 1 à 9, pour le conditionnement de substances kératiniques, en particulier de fibres kératiniques et notamment de fibres kératiniques humaines, telles que les cheveux.
